# EUROPEAN PATENT APPLICATION

(11) **EP 2 425 828 A2**
(43) Date of publication of application: **07.03.2012**
(21) Application number: 11250640.7
(22) Date of filing: 08.07.2011
(51) Int. Cl.: A61K 9/70, A61K 31/445

(54) **Films for delivery of a therapeutic agent**

(30) Priority: 08.07.2010 US 362483 P; 30.06.2011 US 173718
(71) Applicant: Tyco Healthcare Group LP, New Haven, CT 06511 (US)
(72) Inventor: Stopek, Joshua, Guilford, CT 06437 (US); Broom, Daniel, Branford, CT 06405 (US); Elachchabi, Amin, Hamden, CT 06518 (US); Ebersole, Garrett, Southington, CT 06489 (US)
(74) Representative: Soames, Candida Jane

(57) **Abstract**

The present disclosure relates to self-supporting films for delivery of a therapeutic agent containing at least one hydrophobic polymer and at least one therapeutic agent. Methods of forming the multilayer films are also disclosed.

## Description

### CROSS-REFERENCE TO RELATED APPLICATIONS

This application claims the benefit of and priority to U.S. Provisional Patent Application No. 61/362,483, the entire disclosure of which is incorporated by reference herein.

### BACKGROUND

### Technical Field

The present disclosure describes films for delivery of a therapeutic agent, and more particularly, films for delivery of a therapeutic agent containing at least one hydrophobic polymer and at least one water-soluble therapeutic agent.

### Background of Related Art

Delivery of a therapeutic agent through the use of implantable medical devices is described in a wide variety of manners. Existing methods of such delivery of a therapeutic agent predominantly focus on the use of water-soluble drugs and hydrophilic polymers to form thin surface coatings positioned on a surface of a medical device which provide limited therapeutic payloads.

In addition, highly water-soluble drugs may be difficult to formulate for controlled release in that highly water-soluble drugs may offer limited solubility in the organic systems particularly useful with hydrophobic or water-insoluble drug carriers, i.e., hydrophobic polymers. Limited solubility of the highly water-soluble drugs may further lead to poor encapsulation efficiencies of the drug and limited therapeutic payload on an implantable device. Such hydrophilic drugs need a sufficient water diffusion barrier to sustain release. Current systems are challenged from a drug payload and sustained release standpoint including offering therapeutic benefits.

It would be beneficial to provide films for delivery of a therapeutic agent which displays enhanced therapeutic payload.

### SUMMARY

A multilayer film is provided herein including a first layer including a first hydrophobic polymer; a third layer including a third hydrophobic polymer; and a second layer disposed between the first layer and the third layer, the second layer including a second hydrophobic polymer and a water-soluble therapeutic agent. The first, second, or third hydrophobic polymer may be selected from aliphatic polyesters, polyhydroxyalkanoates, polyorthoesters, polyurethanes, polyanhydrides, polymer drugs, and combinations thereof. More specifically, the aliphatic polyester may be selected from lactide; glycolide; trimethylene carbonate; Ä-valerolactone; â-butyrolactone; ã-butyrolactone; å-decalactone; hydroxybutyrate; hydroxyvalerate; 1,4-dioxepan-2-one; 1,5-dioxepan-2-one; 6,6-dimethyl- 1,4-dioxan-2-one; 2,5-diketomorpholine; pivalolactone; á, á diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and homopolymers and copolymers and combinations thereof. In one embodiment, at least one of the first, second or third hydrophobic polymer include a copolymer of about 10 weight % glycolide and about 90 weight % ε -caprolactone.

The multilayer film includes at least one water-soluble therapeutic agent which may be selected from antimicrobial agents, anesthetics, analgesics, anticancer agents, angiogenic agents, antiseptics, antibiotics, fibrotic agents, antimitotics, chelating agents, peptides, proteins, DNA, RNA, nucleotides, liposomes, blood products, hormones, water-soluble silver salts, growth factors, antibodies, interleukins, cytokines, and combinations thereof. In some embodiments, the at least one water-soluble therapeutic agent is selected from bupivacaine, 5-fluorouracil, cisplatin, methotrexate, and capsaicin. More specifically, the at least one water-soluble therapeutic agent may include bupivacaine hydrochloride. The at least one water-soluble therapeutic agent disclosed herein may have a therapeutic payload of from about 1 mg/cm² to about 50 mg/cm². Alternatively, the multilayer film may include a therapeutic payload of at least 300 mg/cm² of bupivacaine HCI.

In certain embodiments, the multilayer film is combined with a mesh, the mesh having a first surface and a second surface. The mesh may be positioned between the first and the third layer. The first surface of the mesh may also be positioned adjacent at least one of the first and the third layers.
In some embodiments, the multilayer film further includes a collagen film.

In one embodiment, a multilayer film composite is disclosed including a mesh including a collagen film; and a multilayer film containing a first layer including a first hydrophobic polymer, a third layer including a third hydrophobic polymer, and a second layer disposed between the first layer and the third layer, the second layer including a second hydrophobic polymer and at least one water-soluble therapeutic agent. The collagen film may be positioned adjacent at least one of the first and second surfaces of the mesh.

A method of making a multilayer film composite is provided herein, the method including the steps of: providing a collagen film; providing a first solution containing at least one hydrophobic polymer; providing a second solution containing at least one water-soluble therapeutic agent; passing the first solution through a first spray nozzle to generate first droplets of the first solution; depositing the first droplets on the collagen film to form a first layer; passing the second solution through a second spray nozzle to generate second droplets of the second solution; depositing the second droplets on the first layer to form a second layer; and drying the first and second layers to form a multilayer film composite. The method may further include the step of combining the multilayer film composite with a mesh. Additionally, the method may include the step of depositing the first droplets on the second layer to form a third layer.

A method of treating cancer is also disclosed herein. Select cancers which may be treated utilizing the multilayer films disclosed herein include those such as breast cancer, lung cancer, esophageal cancer, gastric cancer, colon cancer, stomach cancer, and brain cancer.

A method of treating post-operative pain is disclosed herein. Post-operative pain associated with surgeries such as hernia repair, hysterectomy, thoracotomy, coronary artery bypass, hemorrhoidectomy, adhesiolysis, breast reconstruction, spine surgery, and joint replacement/repair may be treated utilizing the multilayer film disclosed herein.

### BRIEF DESCRIPTION OF THE DRAWING

These and other characteristics of the present disclosure will be more fully understood by reference to the following detailed description in conjunction with the attached drawings, in which:

FIG. 1 is a diagrammatic illustration of a barrier layer realized as a film, according to one embodiment described in the present disclosure;

FIGS. 2A, 2B, and 2C are cross-sectional views of multilayer films in accordance with embodiments described in the present disclosure;

FIGS. 3A, 3B, 3C, 3D, 3E, and 3F are diagrammatic views of multilayer films in accordance with other embodiments described in the present disclosure;

FIG. 4A is a cross-sectional view of a multilayer film in accordance with yet another embodiment described in the present disclosure;

FIG. 4B is a cross-sectional view of a multilayer film in accordance with yet another embodiment described in the present disclosure;

FIG. 5 is a cross-sectional view of a multilayer film including a discontinuous layer in accordance with an embodiment of the present disclosure;

FIG. 6 is a cross-sectional view of a multilayer film composite in accordance with another embodiment of the present disclosure;

FIG. 7 is a schematic illustration in partial cross-section illustrating one example of how multilayer films according to the present disclosure are formed;

FIG. 8 is a graph illustrating a comparison of the therapeutic payload on a medical device prepared via dip-coating;

FIG. 9 is a graph illustrating a comparison of the therapeutic payload on a medical device prepared via dip-coating and spray-coating;

FIG. 10 is a graph illustrating a comparison of the therapeutic release of bupivicaine HCL from multilaminar and pouch configured medical devices;

FIG. 11 is a graph illustrating a comparison of the therapeutic release of bupivicaine HCL from films of varying thicknesses; and

FIG. 12 is a graph illustrating a comparison of the therapeutic release of bupivicaine HCL from films formed from various oases of a sprayer.

### DETAILED DESCRIPTION

The present disclosure describes films suitable for delivery of a therapeutic agent. The implantable films include at least one hydrophobic polymer and at least one water-soluble therapeutic agent. The films include a high therapeutic payload of the therapeutic agent and may be multilayered. By multilayered film, it should be understood that a multilayered film includes more than one film layer. For example, a multilayered film may include at least two film layers.

The films may form a generally planar configuration displaying a mechanical strength sufficient to maintain the film in the generally planar configuration before, during, and/or after implantation. Although the films do not require an additional substrate for support or to maintain the predetermined configuration, it is envisioned that in some embodiments, the films described herein may also be combined with an implantable medical device, such as a surgical mesh.

In certain embodiments, the multilayer film is combined with a surgical mesh to create a multilayer composite.

The surgical mesh described herein may include porous fabrics made from intertwined filaments. The filaments may extend horizontally and vertically in a manner which produces sections where the filaments cross-over one another creating points of common intersection. The surgical mesh may be woven, non-woven, knitted, or braided. In some embodiments, the filaments may form two-dimensional or three-dimensional meshes. Some examples of two-dimensional and/or three-dimensional mesh substrates may be found in U.S. Patent No. 7,021,086, U.S. Patent No. 6,596,002, and U.S. Patent No. 7,331,199, the entire contents of each of which are incorporated by reference herein.

In certain embodiments, non-textile based meshes may be manufactured utilizing die or laser cutting, injection molding, and electro spinning processes.

Suitable meshes for use in the present disclosure include, for example, a collagen composite mesh such as PARIETEX^{™} Composite Mesh (commercially available from Tyco Healthcare Group LG, d/b/a Covidien). PARIETEX^{™} Composite Mesh is a 3-dimensional polyester weave with a resorbable collagen film bonded on one side. Another suitable mesh includes Parietex Progrip^{™} self-fixating mesh (also commercially available from Covidien). Parietex Progrip ^{™} is a polyester mesh which includes poly lactic acid (PLA) microgrips. Other suitable meshes include those sold under the names PARIETENEO, PARIETEX^{™}, and SURGIPRO^{™} (all commercially available from Covidien); PROLENE^{TM} (commercially available from Ethicon, Inc.); MARLEX®, DULEX®, 3D MAX® mesh, PERFIX® plug, VENTRALEX®, and KUGEL® patch (all commercially available from C.R. Bard, Inc.); PROLlTE ^{™} and PROLITE ULTRA^{™} (all commercially available from Atrium Medical); COMPOSIX®, SEPRAMESH®, and VISILEX® (all commercially available from Davol, Inc.); and DUALMESH®, MYCROMESH®, and INFINIT® mesh (all commercially available from W.L. Gore). Additionally, meshes within the scope and context of this disclosure may include biologic materials such as allografts (e.g., AlloDerm® Regenerative Tissue Matrix from Lifecell), autografts, and xenografts (e.g., PERMACOL^{™} from Covidien). In alternate embodiments, processed/purified tissues may also be employed.

In certain embodiments, Parietex^{™} Composite Mesh or Parietex^{™} Pro-grip may be utilized in accordance with the present disclosure.

Other suitable non-textile based meshes include foams, films, sponges, composites, and combinations thereof.

The thickness of the films may vary depending upon the number of layers in the film and the number of 'passes' during the spraying process (later described). However, the thickness of the films described herein may on average measure between about 0.1 µm to about 3000 µm. In some embodiments, the thickness of the films may measure between about 1.0 µm to about 1000 µm. In still other embodiments, the thickness of the films may measure between about 10 µm to about 500 µm. Film thickness may influence parameters such as mechanical strength of the multilayer films.

The films described herein may also include a high therapeutic payload of at least one therapeutic agent, especially as compared to other known films of equal thickness. For example, in some embodiments, the therapeutic agent may represent from about 0.1 mg/cm² to about 50 mg/cm². In still other embodiments, the therapeutic agent may represent from about 1 mg/cm² to about 25 mg/cm². More specifically, the therapeutic payload of 5-fluorouracil, methotrexate, capsaicin, and bupivacaine hydrochloride is at least 1 mg/cm² and up to about 50 mg/cm². In other embodiments, different therapeutic agents may be present in different amounts.

Unlike coatings or films formed by solvent casting or immersion coating, the therapeutic films described herein are not limited by the solubility of the water-soluble agent and/or the hydrophobic polymer in a common solvent. Rather, the hydrophobic polymer and therapeutic agent of the films do not share a common solvent and thus the water-soluble therapeutic agent is not limited in solubility by the hydrophobic polymer. For example, the films may be formed using at least two solutions, wherein the hydrophobic polymer forms a first solution in a first solvent (e.g., an organic or hydrophobic solvent) and the water-soluble therapeutic agent forms a second solution in a second solvent (e.g., a hydrophilic solvent).

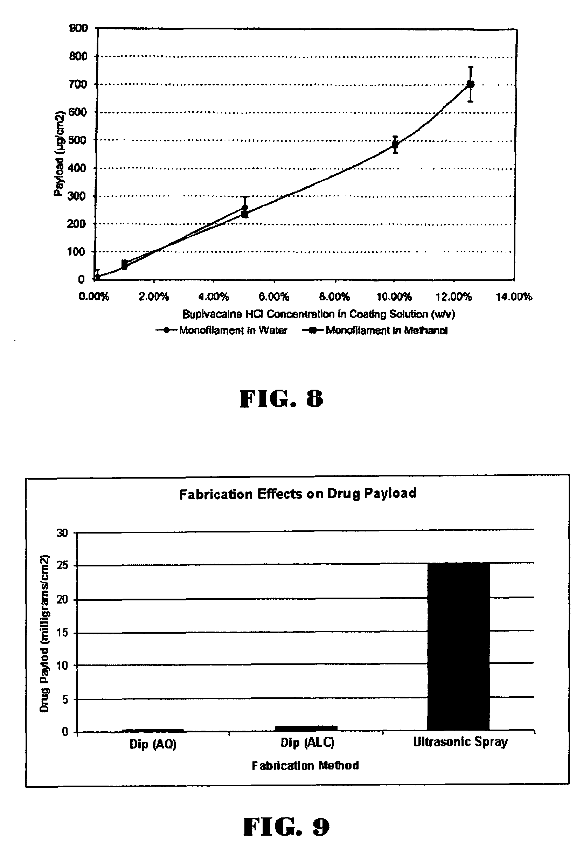

Further, it is believed that spraying films on a medical device (e.g., mesh) compared to other conventional methods, such as dip coating, provides increased loading of the therapeutic agent. As demonstrated in Figure 8 below, the saturation and payload limits for dip coating a bupivacaine hydrochloride (HCI) solution (in water) is 5 % w/v (weight/volume) while dip coating bupivacaine HCI solution (in MeOH) is 12% w/v.

More particularly Figure 8 illustrates the loading limits for a polyester mesh (Parietex^{TM} monofilament mesh) without a polymer film.

Figure 9 illustrates data presented in Figure 8, coating a mesh with bupivacaine HCI utilizing a dip coating method in water (AQ) and in alcohol (ALC), compared to films which have been ultrasonically sprayed as described in Example 5 (PS35-1, PS30-4). The payload limits for the dip coated mesh, AQ (Aqueous, e.g., water) and ALC (Alcohol, e.g., MeOH) do not exceed 1 mg/cm². Conversely, films created using ultrasonic spraying as described herein, can be configured to load up to at least about 25 mg/cm². Drug loading created through ultrasonic spraying of films on mesh, unlike drug loading via dip coating mesh, meets the clinical target payload of >300 mg.

The films ability to include a high therapeutic payload of the therapeutic agent may be a result of the unique manner in which the films may be formed. For example, a film as described herein may be fabricated by passing a first solution containing the hydrophobic polymer and a second solution containing the therapeutic agent through an ultrasonic spray nozzle to form droplets. The droplets may be mixed while falling towards or being deposited onto a substrate, which in certain embodiments, may be a collagen film. In other embodiments, the droplets may be deposited on an insert substrate, such as a silicone sheet, and after drying, the film may be separated from the inert substrate.

The film thickness may be controlled by factors such as the number of applications of the first and second solutions, the length of time/rate of spraying the first and second solutions, polymer solution composition, drug solution composition, flow rate, and use of additives such as viscosity modifiers.

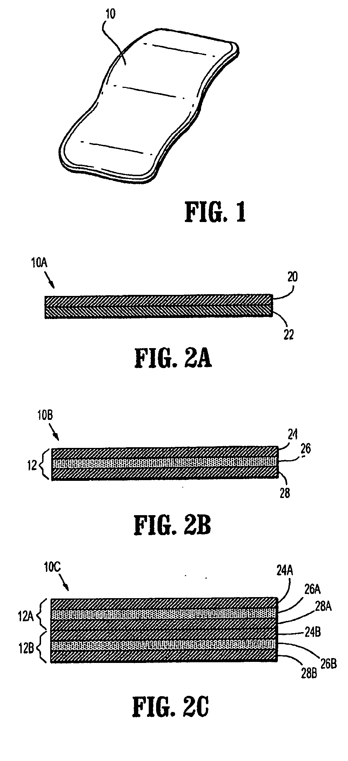

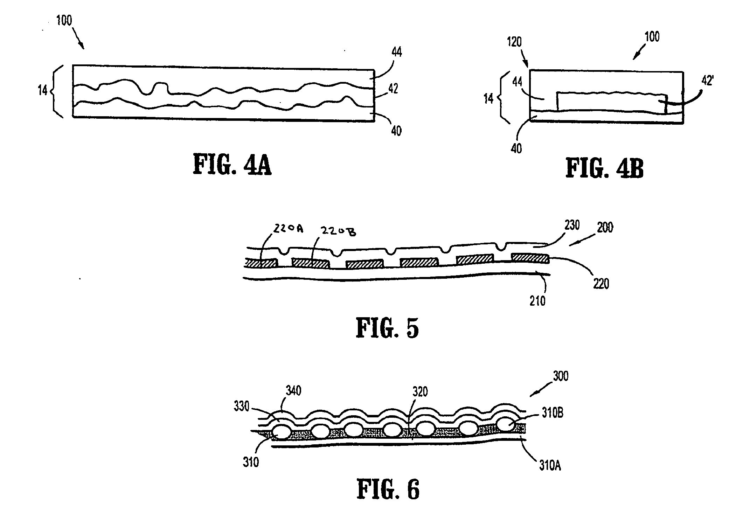

Films of the present disclosure may include continuous or discontinuous films. For example, a continuous film such as those shown in Figures 2A-2C and 4A-4B include at least one single, uninterrupted layer. In another embodiment, films or layers of films may be discontinuous, as illustrated in Figure 5 (later described). Individual layers of the multilayer films may be continuous or discontinuous.

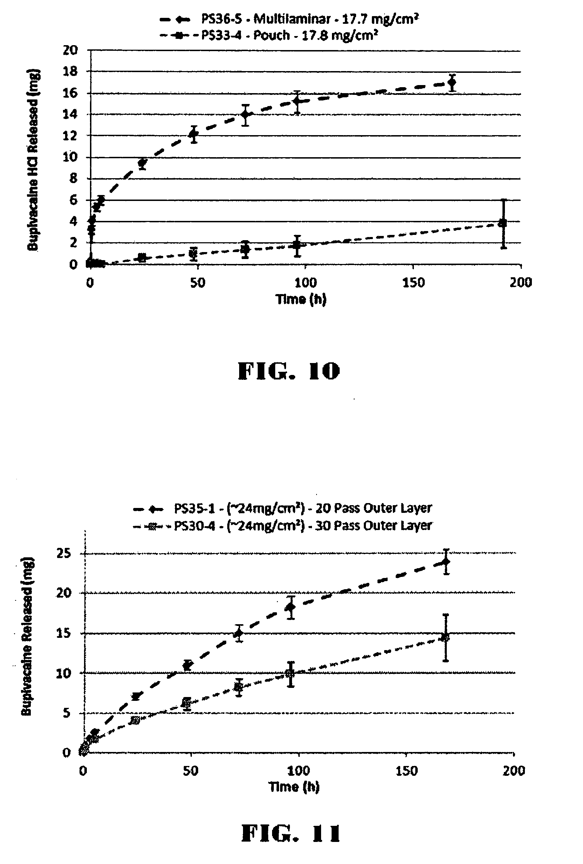

Additionally, the film's construct may control parameters such as drug release and diffusion. In some embodiments the layer containing the therapeutic agent extends to the edge of the multilayer film (See FIG. 4A), in yet alternate embodiments, the layer containing the therapeutic agent does not extend to the edge of the multilayer film, referred to herein as a pouch configuration (See FIG 4B). Release rates may vary depending on the whether or not the therapeutic agent is present at the edge of the multilayer film. Figure 10 illustrates on exemplary embodiment in which the bupivacaine HCI release is slowed down utilizing a pouch configuration. The multilaminar configuration has been formulated to include about 17.7 mg/cm² of bupivacaine HCI, and the pouch configuration has been formulated to include about 17.8 mg/cm² of bupivacaine HCI. Example 7 described herein provides additional information with regards to processing parameters, etc. It should be understood that if the below data was extracted for a longer period of time, total release for the two configurations would be approximately similar.

Film thickness may also play a role in the drug release and diffusion. For example, it has been shown that as film thickness increases, the rate of release for the therapeutic agent decreases or slows down over time. This data is illustrated in Figure 10.

The films created in Figure 11 are described in greater detail in Example 5. Thicker films (e.g., with increased number of passes), have a slower release rate, compared with thinner films. Both films above have been formulated to include about 24 mg/cm² of bupivacaine HCI. It should be understood that if the data was extracted for a longer time point, total release for the different configurations would be approximately similar.

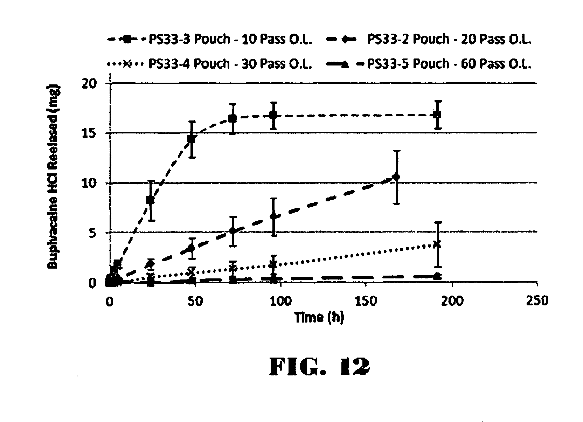

Similarly, for closed-edge samples, increasing the film thickness and/or changing the number of passes per layer alters the release rate of the therapeutic agent. Figure 12 illustrates how increasing the number of passes in each outer layer, changes the release profile and release rate. The release rate slows down as the number of passes in the outer layers increase. Samples shown are exemplary embodiments and depending on rate of release and total release desired, the parameters may be altered to change total release and release rate. It should be understood that if the data was extracted for a longer time point, total release for the different configurations would be approximately similar. Example 6 described herein provides additional information with regards to processing parameters, etc.

In some embodiments, the films include a single layer containing a hydrophobic polymer and a therapeutic agent. A multilayered film may be created by stacking or combining several layers of films containing the hydrophilic polymer and therapeutic agent. In other embodiments, the multilayered films include a first layer containing a hydrophobic polymer and a second layer containing a therapeutic agent. It may be useful to have an exposed layer of a therapeutic agent for providing unidirectional drug delivery.

In other embodiments, the films include a tri-layer structure wherein a third layer containing a therapeutic agent, is positioned between a first layer containing a hydrophobic polymer and a second layer containing the same or a different hydrophobic polymer. The third layer may include a hydrophobic polymer and a therapeutic agent, whereas the first and second layer may include only a hydrophobic polymer. For example, the first and second layers may include a copolymer of about 10 weight % glycolide and about 90 weight % ε-caprolactone; while the third layer includes a copolymer of about 10 weight % glycolide and about 90 weight % ε -caprolactone in combination with bupivacaine hydrochloride.

The tri-layer structure, similar to a sandwich-type structure, may be combined with other films including other single, double, and/or other tri-layer structures. As previously discussed, any of the multilayer films disclosed herein may be combined with mesh. It should be noted that multiple layers may utilize similar or different polymers depending on the intended use as well as drug release profiles. Further, thicker and thinner layers may be created through process variation.

The films may include any hydrophobic polymer suitable for implantation and capable of displaying sufficient mechanical strength to be considered. Certain layers may also include hydrophilic polymers. Some non-limiting examples of suitable absorbable polymers which may be utilized in accordance with the present disclosure include or may be derived from at least one of the following materials: aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

More specifically, for the purpose of this disclosure, aliphatic polyesters include, but are not limited to, homopolymers and copolymers of lactide (including lactic acid, D-,L- and meso lactide); glycolide (including glycolic acid); å-caprolactone; p-dioxanone (1,4-dioxan-2-one); trimethylene carbonate (1,3-dioxan-2-one); alkyl derivatives of trimethylene carbonate; Ä-valerolactone; â-butyrolactone; ã-butyrolactone; å-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; á, á diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

In certain embodiments, the hydrophobic polymers may include homopolymers or copolymers which include lactide, glycolide, dioxanone, trimethylene carbonate, and ε-caprolactone. For example, the therapeutic agents described herein may be combined with copolymers, e.g., random or block copolymers, of lactide and glycolide, or glycolide and ε-caprolactone. Increasing the amount of glycolide may increase the films degradation rate. While increasing the amount of lactide and/or caprolactone may extend the degradation or absorption profile of the film. For example, lactide rich copolymers, e.g., greater than about 50% lactide, may be particularly useful to enhance a particular polymer's solubility, such as glycolide. In particular embodiments, a copolymer of about 10 wt (weight) % glycolide and about 90 wt % ε-caprolactone may be utilized.

Other suitable biodegradable polymers which may be utilized in accordance with the present disclosure include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Additionally, synthetically modified natural polymers such as cellulose and polysaccharide derivatives, including alkyl celluloses, hydroxyalkyl celluloses, cellulose ethers, cellulose esters, nitrocelluloses, and chitosan may be utilized. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses".

In certain embodiments, the hydrophilic polymers may include the first, second, and third layers. A copolymer of about 10 wt % glycolide and about 90 wt % ε -caprolactone may be utilized to form the first, second, and third layers.

In alternate embodiments, at least one layer may include a hydrophobic polymer.

The hydrophobic polymers of the present disclosure may form polymer solutions, which in turn, are passed through an ultrasonic sprayer to create the multilayered films. Polymer solutions for the purpose of this disclosure include suspensions, emulsions, dispersions, and the like. Some non-limiting examples of polar and non-polar solvents suitable for forming the polymer solutions may include chlorinated solvents (e.g., methylene chloride, dichloroethane, perchloroethane, and chloroform), N-methylpyrrolidone, tetrahydrofuran, dimethylformamide, methanol, ethanol, hexanes, acetone, acetic acid, water, saline and combinations thereof. In particular embodiments, the solvent used for the polymer solution may not be the same solvent used to form the therapeutic agent solution. More specifically, the first solution (containing the hydrophobic polymer) may include a chlorinated solvent such as methylene chloride, chloroform, dichloromethane, and perchloroethane. The second solution (containing the water-soluble therapeutic agent) may include a hydrophilic solvent such as methanol, ethanol, water, saline, isopropanol, and acetic acid. The polymer may represent from about 0.5% to about 25% (w/w) in the solution, in embodiments, between about 1% and about 10% (w/w) in the solution.

In addition, the polymeric solutions and/or the therapeutic solutions may include at least one optional ingredient such as emulsifiers, viscosity enhancers, dyes, pigments, fragrances, pH modifiers, wetting agents, plasticizers, antioxidants, foaming agents, amphiphilic compounds, and the like. For instance, the solutions may include a foaming agent or pore former to induce porosity of the film. In another example, the solutions may include amphiphilic compounds to increase water diffusion of the film. The optional ingredients may represent up to about 10% (w/w) of the polymer solution.

Tissue reactive chemistries may also be added to the multilayered films of the present disclosure. Suitable tissue reactive chemistries include, for example, reactive silicones, isocyanates, N-hydroxy succinimides ("NHS"), cyanoacrylates, aldehydes (e.g., formaldehydes, glutaraldehydes, glyceraldehydes, and dialdehydes), and genipin. As used herein, succinimides also include sulfosuccinimides, succinimide esters and sulfosuccinimide esters including N-hydroxysuccinimide ("NHS"), N-hydroxysulfosuccinimide ("SNHS"), N-hydroxyethoxylated succinimide ("ENHS"), N-hydroxysuccinimide acrylate, succinimidyl glutarate, n-hydroxysuccinimide hydroxybutyrate, combinations thereof, and the like.

The term "therapeutic agent" as used herein, is used in its broadest sense and includes any substance or mixture of substances that provides a beneficial, therapeutic, pharmacological, and/or prophylactic effect. The agent may be a drug which provides a specific pharmacological effect.

The term "drug" is meant to include any agent capable of rendering a therapeutic effect, such as antimicrobial agents, anesthetics, analgesics, anticancer agents, angiogenic agents, fibrotic agents, antimitotics, antibiotics, anti-inflammatory agents, chelating agents, peptides, proteins, DNA, RNA, nucleotides, liposomes, blood products, hormones, water-soluble or insoluble silver salts or particles, growth factors, antibodies, interleukins, cytokines, and the like. The term "drug' is also intended to include any compound that affects or participates in tissue growth, cell growth, cell differentiation, anti-adhesion of tissue; or a compound that may be able to invoke a biological action such as an immune response; or a compound that could play any other role in one or more biological processes. Examples of classes of bioactive agents, which may be utilized in accordance with the present disclosure include, for example, anti-adhesives, antimicrobials, analgesics, antipyretics, anesthetics (e.g., local, regional, and systemic), antiepileptics, antihistamines, anti-inflammatories, cardiovascular drugs, diagnostic agents, sympathomimetics, cholinomimetics, antimuscarinics, antispasmodics, hormones, growth factors, muscle relaxants, adrenergic neuron blockers, antineoplastics, immunogenic agents, immunosuppressants, gastrointestinal drugs, diuretics, steroids, lipids, phosphoryl cholines, lipopolysaccharides, polysaccharides, platelet activating drugs, clotting factors, and enzymes. It is also intended that combinations of bioactive agents may be used.

Other bioactive agents, include: anti-fertility agents; parasympathomimetic agents; psychotherapeutic agents; tranquilizers; decongestants; sedative hypnotics; sulfonamides; sympathomimetic agents; vaccines; vitamins; antimalarials; anti-migraine agents; anti-parkinson agents such as L-dopa; anti-spasmodics; anticholinergic agents (e.g., oxybutynin); antitussives; bronchodilators; cardiovascular agents such as coronary vasodilators and nitroglycerin; alkaloids; analgesics; narcotics such as codeine, dihydrocodeinone, meperidine, morphine and the like; non-narcotics such as salicylates, aspirin, acetaminophen, d-propoxyphene and the like; opioid receptor antagonists such as naltrexone and naloxone; anti-cancer agents; anticonvulsants; anti-emetics; antihistamines; anti-inflammatory agents such as hormonal agents, hydrocortisone, prednisolone, prednisone, non-hormonal agents, allopurinol, indomethacin, phenylbutazone, and the like; prostaglandins and cytotoxic drugs; chemotherapeutics; estrogens; antibacterials; antibiotics; anti-fungals; anti-virals; anticoagulants; anticonvulsants; antidepressants; and immunological agents.

Other examples of suitable bioactive agents, which may be included in the film include, for example, viruses and cells; peptides, polypeptides and proteins, as well as analogs, muteins, and active fragments thereof; immunoglobulins; antibodies; cytokines (e.g., lymphokines, monokines, chemokines); blood clotting factors; hemopoietic factors; interleukins (e.g., IL-2, IL-3, IL-4, IL-6); interferons (e.g., β-IFN, α-IFN and y-IFN); erythropoietin; nucleases; tumor necrosis factor; colony stimulating factors (e.g., GCSF, GM-CSF, MCSF); insulin; anti-tumor agents and tumor suppressors; blood proteins such as fibrin, thrombin, fibrinogen, synthetic thrombin, synthetic fibrin, synthetic fibrinogen; gonadotropins (e.g., FSH, LH, CG, etc.); hormones and hormone analogs (e.g., growth hormone); vaccines (e.g., tumoral, bacterial and viral antigens); somatostatin; antigens; blood coagulation factors; growth factors (e.g., nerve growth factor, insulin-like growth factor); bone morphogenic proteins; TGF- β; protein inhibitors; protein antagonists; protein agonists; nucleic acids such as antisense molecules, DNA, RNA, and RNAi; oligonucleotides; polynucleotides; and ribozymes.

In embodiments, the bioactive agent may include at least one of the following drugs, including combinations and alternative forms of the drugs such as alternative salt forms, free acid form, free base forms, pro-drugs, and hydrates. Specific agents within these classes are within the purview of those skilled in the art and are dependent upon such factors as, for example, the type of device in which it is utilized and the tissue being treated. Thus, for example, local anesthetics such as bupivacaine (which may be sold under Marcain^{TM}, Marcaine^{TM}, Sensorcaine^{TM} and Vivacaine^{TM} all by AstraZeneca), levobupivacaine (sold under Chirocaine^{TM} by AstraZeneca), ropivacaine, lidocaine, and the like, may be used alone or in combination for treatment of pain or for anesthetic purposes. In embodiments, antimicrobial agents such as triclosan, also known as 2,4,4'-trichloro-2'-hydroxydiphenyl ether; chlorhexidine and its salts, including chlorhexidine acetate, chlorhexidine gluconate, chlorhexidine hydrochloride, and chlorhexidine sulfate; silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine; polymyxin; tetracycline; aminoglycosides such as tobramycin and gentamicin; rifampicin; bacitracin; neomycin; chloramphenicol; miconazole; quinolones such as oxolinic acid, norfloxacin, nalidixic acid, pefloxacin, enoxacin and ciprofloxacin; penicillins such as oxacillin and pipracil; nonoxynol 9; fusidic acid; and cephalosporins; may also be used alone or in combination for the treatment of microbial growth. In addition, antimicrobial proteins and peptides, such as lactoferrin and lactoferricin B, and antimicrobial polysaccharides, such as fucans and derivatives thereof, may be included as a bioactive agent in the present disclosure to kill or prevent microbial growth. And anti-adhesive agents may be used to prevent adhesions from forming between the coated medical device and the surrounding tissues. Some examples of these agents include, but are not limited to, poly(vinyl pyrrolidone), carboxymethyl cellulose, hyaluronic acid, alginate, collagen, polyethylene glycol, polyethylene oxide, polypropylene glycol, poly vinyl alcohols, poly acrylic acid, styrene sulfonic acid, polyhydroxyethylmethylacrylate, (pHEMA), and phospholipid vinyls; acrylic polymers such as sodium polyacrylate, polyethylacrylate, and polyacrylamide, polypropylene oxide, phosphorylcholine functional acrylates and methacrylates; homopolymers and combinations thereof.

Some specific non-limiting examples of water-soluble drugs that may be used in the present films include, lidocaine, bupivacaine hydrochloride, capsaicin, fluorouracil, cisplatin, methotrexate, tetracaine, procaine, dibucaine, sirolimus, taxol, chlorhexidine, polyhexamethylene, thiamylal sodium, thiopental sodium, ketamine, flurazepam, amobarbital sodium, phenobarbital, bromovalerylurea, chloral hydrate, phenytoin, ethotoin, trimethadione, primidone, ethosuximide, carbamazepine, valproate, acetaminophen, phenacetin, aspirin, sodium salicylate, aminopyrine, antipyrine, sulpyrine, mepirizole, tiaramide, perixazole, diclofenac, anfenac, buprenorphine, butorphanol, eptazocine, dimenhydrinate, difenidol, dlisoprenaline, chlorpromazine, levomepromazine, thioridazine, fluphenazine, thiothixene, flupenthixol, floropipamide, moperone, carpipramine, clocapramine, imipramine, desipramine, maprotiline, chlordiazepoxide, clorazepate, meprobamate, hydroxyzine, saflazine, ethyl aminobenzoate, chlorphenesin carbamate, methocarbamol, acetylcholine, neostigmine, atropine, scopolamine, papaverine, biperiden, trihexyphenidyl, amantadine, piroheptine, profenamine, levodopa, mazaticol, diphenhydramine, carbinoxamine, chlorpheniramine, clemastine, aminophylline, choline, theophylline, caffeine, sodium benzoate, isoproterenol, dopamine, dobutamine, propranolol, alprenolol, bupranolol, timolol, metoprolol, procainamide, quinidine, ajmaline, verapamil, aprindine, hydrochlorothiazide, acetazolamide, isosorbide, ethacrynic acid, captopril, enalapril, delapril, alacepril, hydralazine, hexamethonium, clonidine, bunitrolol, guanethidine, bethanidine, phenylephrine, methoxamine, diltiazem, nicorandil, nicametate, nicotinic-alcohol tartrate, tolazoline, nicardipine, ifenprodil, piperidinocarbamate, cinepazide, thiapride, dimorpholamine, levallorphan, naloxone, hydrocortisone, dexamethasone, prednisolone, norethisterone, clomiphene, tetracycline, methyl salicylate, isothipendyl, crotamiton, salicylic acid, nystatin, econazole, cloconazole, vitamin B₁ , cycothiamine, vitamin B₂, vitamin B₃, vitamin B₅, vitamin B₆, vitamin B₇, vitamin B₉, vitamin B₁₂, vitamin C, nicotinic acid, folic acid, nicotinamide, calcium pantothenate, pantothenol, panthetin, biotin, ascorbic acid, tranexamic acid, ethamsylate, protamine, colchicine, allopurinol, tolazamide, glymidine, glybuzole, metoformin, buformin, orotic acid, azathioprine, lactulose, nitrogen mustard, cyclophophamide, thio-TEPA, nimustine, thioinosine, fluorouracil, tegafur, vinblastine, vincristine, vindesine, mitomycin C, daunorubicin, aclarubicin, procarbazine, cisplatin, methotrexate, benzyl penicillin, amoxicillin, penicillin, oxycillin, methicillin, carbenicillin, ampicillin, cefalexin, cefazolin, erythromycin, kitasamycin, chloramphenicol, thiamphenicol, minocycline, lincomycin, clindamycin, streptomycin, kanamycin, fradiomycin, gentamycin, spectinomycin, neomycin, vanomycin, tetracycline, ciprofloxacin, sulfanilic acid, cycloserine, sulfisomidine, isoniazid, ethambutol, acyclovir, gancyclovir, vidabarine, azidothymidine, dideoxyinosine, dideoxycytosine, morphine, codeine, oxycodone, hydrocodone, cocaine, pethidine, fentanyl, polymeric forms of any of the above drugs and any combinations thereof.

The water-soluble drug may not need to be converted to a salt form, i.e., tetracycline hydrochloride. In some embodiments, the therapeutic agent may include an anesthetic, e.g., bupivacaine, lidocaine, benzocaine, and the like.

The multilayer films described herein may be used in treating cancers not limited to those such as breast cancer, lung cancer, esophageal cancer, gastric cancer, colon cancer, stomach cancer, and brain cancer. Suitable water-soluble therapeutic agents which may be used in treating cancer include, but are not limited to 5-fluorouracil, methotrexate, cisplatin, daunorubucub, mitoxantrone, and carboplatin.

The multilayer films described herein may also be used in treating post-operative pain. Post-operative pain may be commonly associated with procedures such as inguinal and ventral hernia repair, hysterectomy, thoracotomy, coronary artery bypass, hemorrhoidectomy, adhesiolysis, breast reconstruction, spine surgery, and joint repair/replacement. Suitable water-soluble therapeutic agents which may be used in treating post-operative pain include, but are not limited to lidocaine hydrochloride, mepivacaine hydrochloride, bupivacaine hydrochloride, and capsaicin. Therapeutic payloads for these water-soluble drugs are described herein.

In particular embodiments, multilayer films may include a copolymer of about 10 weight % glycolide and about 90 weight % ε-caprolactone in combination with a therapeutic agent such as bupivacaine hydrochloride, capsaicin, or 5-fluorouracil.

The water-soluble therapeutic agents may be combined with any suitable solvent to form a therapeutic solution. In some embodiments, the solvent for the therapeutic agent is not a cosolvent for the hydrophobic polymer, that is to say the therapeutic agent is not miscible in the solvent utilized for the hydrophobic polymer, and the hydrophobic polymer is not miscible in the solvent for the therapeutic agent.

The water-soluble therapeutic agent may form a solution at a concentration ranging from about 1 microgram/mL to about 500 mg/mL. In certain embodiments, the concentration of the therapeutic solution may range from about 1 mg/mL to about 200 mg/mL. In still other embodiments, the concentration of the therapeutic solution may range from about 10 mg/mL to about 100 mg/mL. By solution, the therapeutic preparation is intended to include suspensions, emulsions, dispersions, and the like.

As discussed briefly above, the therapeutic solution and the polymer solution may be passed through an ultrasonic spray nozzle to form the multilayer films described herein. Ultrasonic sprayers include ultrasonic spray nozzles which may be used to generate vibrations leading to atomization of the solutions. The sprayer body includes three principal sections: front horn, the atomizing section; rear horn, the rear section; and a section including a pair of disc-shaped piezoelectric transducers. Working in unison, these three elements provide means for creating the vibration required to atomize the solutions delivered to the nozzle surface. The solutions enter through a fitting on the rear horn, passes through a tube, and then a central axis of the front horn. Finally, the solution reaches the atomizing surface of the nozzle where atomization takes place. Piezoelectric transducers convert electrical energy provided by an external power source into high-frequency mechanical motion or vibration. The solution absorbs the underlying vibration energy and generates capillary waves. When the amplitude of the capillary waves exceeds a critical value, the waves collapse ejecting small droplets of the solutions.

The ultrasonic sprayer nozzle may include a variety of controls which may be adjusted to alter the characteristics of the films described herein. Some non-limiting examples include: vibration frequency, operational power; solution flow rates, nozzle speed, and length of movement of the nozzle. In forming the films described herein, the sprayer nozzle may vibrate at a frequency ranging from about 20 kHz to about 240 kHz, in some embodiments, a 120 kHz nozzle may be used. The nozzle may operate at a power ranging from about 2 to about 10 watts. In some embodiments, the sprayer nozzle may vibrate at a frequency of about 48 kHz and operate at a power of about 6 watts.

In certain embodiments, the ultrasonic spray nozzles may be movable. The nozzle may move a speed ranging from about 1 mm/sec to about 200 mm/sec. In other embodiments, the nozzle speed may range from about 50 mm/sec to about 150 mm/sec. In addition, the height of the movable nozzles may range from about 30 mm to about 60 mm from the inert substrate. As the nozzle moves across the substrate, creating the multilayer film, each complete movement translating across the substrate is referred to as a 'pass'. For example, if the multilayer film is created by the nozzle passing across the substrates five times, the spray nozzle has taken five passes.

Also, the flow rate of the solutions passed through the sprayer nozzle may vary within the range of about 0.1 mL/min to about 5 mL/min. In embodiments, the flow rate of the solutions may be within the range of about 0.5 mL/min and about 2.0 mL/min. The flow rate may be different for each of the polymer solution and the therapeutic solutions. It is envisioned that each of the sprayer controls may be individually adjusted for each of the different solutions being passed therethrough.

Multilayer films of the present disclosure may be sprayed onto an inert substrate which may include a release liner substrate utilized for fabrication only. The inert substrate may be separated from the film prior to packaging or conversely, the substrate may be packaged with the film and removed prior to implantation. In other embodiments, a mesh may be positioned on an inert substrate, and the multilayer film may be sprayed directly onto mesh, creating a multilayer composite film. In other embodiments, the inert substrate is part of the implantable therapeutic multilayer composite film.

In yet alternate embodiments, the mesh may be provided with a film backing, such as collagen. The multilayer film may be directly sprayed onto the collagen film backing, which then may be subsequently combined with a mesh, creating a multilayer film composite. For example, the polymer film containing a multilayer film may be combined with a mesh using methods discussed hereinbelow.

Films may be dried at ambient (about 25°C) or elevated temperatures and humidity. Depending on film thickness/number of layers, the films may dry in from about 1 minute to about 24 hours.

Multilayer films of the present disclosure may be combined with meshes described herein using methods including, but not limited to, adhesives, glues, solvent welding, spot welding, solvent casting, melt pressing, heat staking, and the like. In other embodiments, the multilayer film may be formed directly on the mesh.

Turning now to the figures, FIG. 1 illustrates film 10 including a hydrophobic polymer and a therapeutic agent contained in a single layer. The film 10 maintains flexibility to the extent it can be handled without tearing prior to implantation and can adjust to various amounts of force when implanted.

FIGS. 2A, 2B, and 2C illustrate side views of multilayer films. For example, in FIG. 2A, film 10A is shown including first layer 20 and second layer 22. First layer 20 includes at least one hydrophobic polymer and second layer 22 includes at least one water-soluble therapeutic agent. In some embodiments, the therapeutic agent and/or the hydrophobic polymer may be combined in either first layer 20 or second layer 22.

FIG. 2B shows multilayer film 10B displaying tri-layer structure 12 including first layer 24, second layer 26, and third layer 28 with a therapeutic agent positioned between two polymer layers. First layer 24 may include at least one hydrophobic polymer and second layer 26 may include at least one water-soluble therapeutic agent. Third layer 28 may include the same or different hydrophobic polymer as included in first layer 24. For example, in some embodiments, second layer 26 may include a therapeutic agent, such as bupivacaine, and the first and third layers may include the same hydrophobic polymer material, e.g., poly(glycolide-co-caprolactone). In another example, all three layers may include the same polymer material, e.g., poly(glycolide-co-caprolactone), and second layer 26 may also include a highly water-soluble therapeutic agent, e.g., bupivacaine.

FIG. 2C illustrates two tri-tayer structure 12A and 12B (as shown in FIG. 2B) stacked on top of each other to form multilayer film 10C. First tri-layer structure 12A includes first layer 24A, second layer 26A, and third layer 28A with a therapeutic agent positioned in second layer 26A between the two polymer layers, first and third layers 24A and 28A. Second tri-layer structure 12B includes fourth layer 24B, fifth layer 26B, and sixth layer 28B with a therapeutic agent positioned in fifth layer 26B between the two polymer layers, fourth and sixth layers 24A and 28A. Although shown as a sandwich-like structure in FIG. 2B, tri-layer structures 12A and 12B may include any conceivable combination of the polymer materials and therapeutic agents described herein.

When stacked, film 10C may include increased payload of the therapeutic agent without compromising mechanical properties. It is envisioned that more than two tri-layer structures 12 may be stacked on top of each other to form the films. In embodiments, 2 to about 25 of the tri-layer structures may be stacked on top of each other to form the multilayer film.

Also, the thickness of each of the individual layers in the multilayer films may control the release of the therapeutic agent from the film. For example, increasing the thickness of only the polymer layer may decrease the rate at which the therapeutic agent may be released into the surrounding tissue following implantation. Conversely, decreasing the thickness of only the polymer layer may increase the rate at which the therapeutic agent may be released.

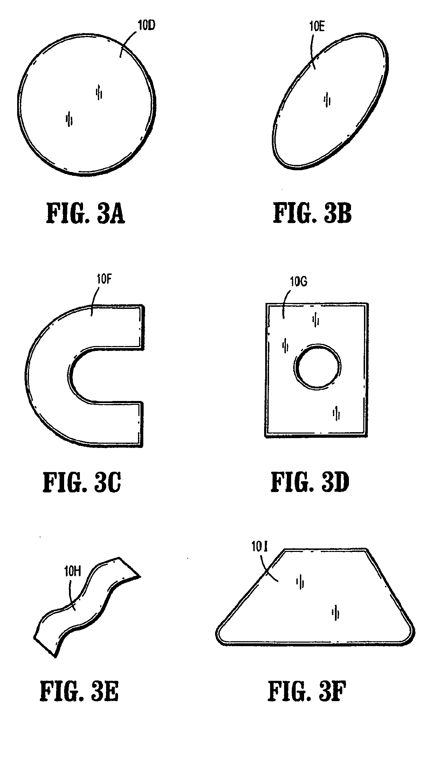

FIGS. 3A-3F show additional embodiments or configurations of films 10. The embodiments include film10D in a circular configuration, film10E in an oval configuration, film10F in a U-bend configuration, film10G in a square configuration having a circular aperture, film10H in a wave configuration, and film10I in an irregular shape configuration. Each of the configurations of films10D through 101 represents different types of configurations. The configurations illustrated are by no means the only possible configurations for film 10. One of ordinary skill in the art will appreciate that the specific shape or configuration of film 10 can vary as desired and that the shapes and configurations depicted in FIG. 1 and FIGS. 3A through 3F are illustrative of only a small number of possible shapes and configuration.

As shown in FIGS. 4A-4B, the multilayer film 100 displays a tri-layer structure 14 including a first layer 40, third layer 44, and second layer 42 disposed therebetween the first and third layer 40 and 44. The second layer includes a water-soluble therapeutic agent and a hydrophobic polymer, while the first and third layers each include a hydrophobic polymer. The hydrophobic polymers may be the same or different in each layer. However, unlike the multiple layers shown in FIGS. 2A-2C, the topography of each of the three layers 40, 42, and 44 may be different throughout the length of the film. Because the films may be formed using an ultrasonic sprayer nozzle, the film layers may be formed from a plurality of droplets which, in some embodiments, may be deposited onto the inert substrate in any random amount.

It will be understood that FIG. 4B is a similar embodiment to FIG. 4A and therefore will only be described with respect to the differences therebetween. The multilayer film 100 includes a tri-layer structure 14, however the second, middle layer 42' does not extend to the outer edge 120 of the film. By controlling the distance of the second layer (containing a therapeutic agent) to the outer edge 120 the release of the therapeutic agent may be altered/controlled.

An alternate embodiment of a tri-layer film having a discontinuous layer is illustrated in FIG. 5. The multilayer film 200 includes a first, continuous layer 210; a third, continuous layer 230; and a second discontinuous layer 220, disposed therebetween. The discontinuous layer 220 may be created using a screen or a template, or a pre-programmed spray pattern. Although the discontinuous layer is illustrated as the second layer, it should be understood that the discontinuous layer may be any of the layers of the multilayer film. The discontinuous layer 220 may include the same or different polymers and/or therapeutic agents. For example, the discontinuous layer 220 may include first sections including a first copolymer and a first therapeutic agent 220a, and second sections including a second copolymer and a second, different therapeutic agent 220b.

In certain embodiments, meshes may be combined with multilayer films of the present disclosure to create a multilayer film composite. The mesh includes a first surface and a second surface. At least one of the first surface or the second surface of the mesh may be positioned adjacent at least one of the first or third layers of the multilayer film. In alternate embodiments, the mesh may be positioned between various layers of the multilayer film. For example, the mesh may be positioned between the first and second layer of the multilayer film.

FIG. 6 illustrates a multilayer film composite in accordance with the present disclosure. The multilayer composite 300, includes collagen backed mesh 310, such as Parietex^{TM} Composite Mesh (PCO) having a multilayer film according to the present disclosure. The mesh structure 310B includes a collagen film 310A on a first surface thereof. The multilayer film disposed on the PCO mesh includes a first, discontinuous layer 320, a second continuous layer 330 and a third, continuous layer 340. As illustrated, the first layer 320 is disposed directly on the collagen film, between the fibers of the mesh 310b. The discontinuous layer 320 may be created using methods described above. The second layer 330 is deposited on mesh 310B and the first layer 320. The third layer 340 is subsequently deposited on the second layer 330.

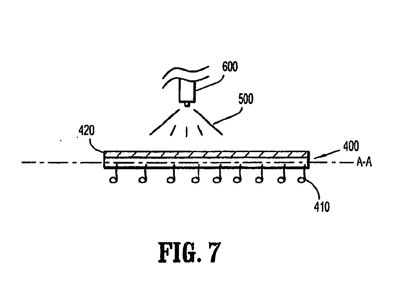

FIG. 7 schematically illustrates the process of spray coating Parietex Progrip^{TM} self-fixating mesh 400. The mesh 400 includes grip members 410 projecting from a first surface of the mesh 400. The grip members extend generally perpendicular to a longitudinal axis A-A of the mesh 400. The mesh 400 also includes a collagen backing 420 positioned on a second surface of the mesh 400. As illustrated a sprayer 600 is dispensing a solution 500 onto the surface of the collagen backing. The sprayer 600 may continue to dispense solution 500 until the multilayer film has been created.

### EXAMPLE 1

A first polymer solution and a second therapeutic solution were provided. The first polymer solution contained 30mg/mL poly(glycolide-co-caprolactone) dissolved in methylene chloride. The second therapeutic solution contained 100 mg/ml of bupivacaine dissolved in methanol.

Both solutions were fed into an ultrasonic sprayer and passed through at least one ultrasonic spray nozzle. The two solutions were kept separate from each other until atomized at the tip of the nozzle. The nozzle vibrated at a frequency of 48 kHz and was operated at 6 watts. The solutions were passed through the nozzle at a flow rate of about 1 mL/min while the nozzle was moving at a rate of 100 mm/sec at heights ranging from 15 mm to 60 mm.

The atomized solutions formed droplets that mixed when the droplets fell from the tip of the nozzle onto an inert sheet of silicone. A nitrogen curtain kept at a pressure of 1.0 kPa surrounded the falling droplets.

The droplets deposited onto the silicone sheet were dried and then separated from the silicone sheet. The sheets were then sterilized and packaged.

### EXAMPLE 2

A first polymer solution and a second therapeutic solution were provided. The first polymer solution contained 3% w/v poly(glycolide-co-caprolactone) dissolved in methylene chloride. The second therapeutic solution contained 100 mg/mL of bupivacaine dissolved in methanol.

The first and second solutions were fed into an ultrasonic sprayer and passed through first and second ultrasonic spray nozzles, respectively. The nozzles vibrated at a frequency of 48 kHz and were operated at 6 watts. The solutions were passed through the nozzles at a flow rate of about 1 mL/min while the nozzles were moving at a rate of 100 mm/sec at heights ranging from 30 mm to 60 mm. A nitrogen curtain kept at a pressure of 1.0 kPa surrounded the falling droplets.

The first polymer solution was passed through the first spray nozzle to form droplets that were deposited onto the silicone sheet to form a first polymer layer. The thickness of the polymer layer was controlled by the number of applications of the coating solution.

Then the first polymer solution and the second therapeutic solution were passed through the first and second spray nozzles, respectively, to form droplets which mixed when the droplets fell from the tip of the nozzles onto the first polymer layer previously formed on the sheet of silicone. This formed a second layer which included both the polymer and therapeutic agent.

Then the first polymer solution was passed through the first spray nozzle again while the flow rate of the second therapeutic solution was stopped. Droplets of the first polymer solution were formed and deposited onto the second layer previously formed on top of the first layer on the silicone sheet to form a tri-layer structure.

The tri-layer structure was then exposed to increased heat and compression to dry and join the tri-layer structure. The film was subsequently combined with a mesh using an adhesive to create a multilayer composite. The multilayer composite was then sterilized and packaged.

### EXAMPLE 3

A first polymer film and a second polymer film were formed containing poly(glycolide-co-caprolactone). The first polymer film was positioned on a silicone sheet beneath an ultrasonic sprayer nozzle.

A therapeutic solution was formed containing 100 mg/mL of bupivacaine dissolved in ethanol. The therapeutic solution was fed into the ultrasonic sprayer and passed through an ultrasonic sprayer nozzle to form droplets that were deposited onto the preformed first polymer film to form an intermediate therapeutic layer.

The second polymer film was positioned on the intermediate therapeutic layer to form a tri-layer structure. The tri-layer structure was sprayed with a binding agent to bind the three layers together. The tri-layer structure was then exposed to heat and/or compression to complete the binding process.

### EXAMPLE 4

A first polymer solution and a second therapeutic solution were provided. The first polymer solution contained 3% w/v poly(glycolide-co-caprolactone) dissolved in methylene chloride. The second therapeutic solution contained 50mg/mL of bupivacaine dissolved in ethanol.

The first and second solutions were fed into an ultrasonic sprayer and passed through first and second ultrasonic spray nozzles, respectively. The nozzles vibrated at a frequency of 48 kHz and were operated at 6 watts. The solutions were passed through the nozzles at a flow rate of about 1 ml/min while the nozzles were moving at a rate of 100 mm/sec at heights ranging from 30 mm to 60 mm. An air curtain kept at a pressure of 1.0 kPa surrounded the falling droplets.

The first polymer solution was passed through the first spray nozzle to form droplets that were deposited onto a collagen film to create a first polymer layer. The thickness of the polymer layer was controlled by the number of applications of the coating solution.

Then the first polymer solution and the second therapeutic solution were passed through the first and second spray nozzles, respectively, to form droplets which mixed when the droplets fell from the tip of the nozzles onto the first polymer layer previously formed on the collagen film. This formed a second layer which included both the polymer and therapeutic agent.

Then the first polymer solution was passed through the first spray nozzle again while the flow rate of the second therapeutic solution was stopped. Droplets of the first polymer solution were formed and deposited onto the second layer previously formed on top of the first layer on the collagen film to form a tri-layer structure.

The tri-layer structure was then combined with a mesh and subsequently exposed to increased heat and compression to dry and join the tri-layer structure to the mesh. The multilayer composite was then sterilized and packaged.

### EXAMPLE 5

A first polymer solution and a second polymer solution were provided. The first polymer solution contained 30 mg/mL poly(glycolide-co-caprolactone) dissolved in dichloromethane. The second polymer solution contained 30 mg/mL of bupivacaine and 30 mg/mL poly(glycolide-co-caprolactone) dissolved in dichloromethane and methanol (7:3 ratio).

The first and second solutions were fed into an ultrasonic sprayer and passed through an ultrasonic spray nozzle. The nozzle vibrated at a frequency of 48 kHz and was operated at 6 watts. The solutions were passed through the nozzles at a flow rate of about 2 mL/min while the nozzle was moving at a rate of 100 mm/sec at a height of 30 mm. A nitrogen curtain kept at a pressure of 1.0 kPa surrounded the falling droplets.

The first polymer solution was passed through a first spray nozzle to form droplets that were deposited onto a collagen sheet to form a first, outer polymer layer. The thickness of the polymer layer was controlled by the number of applications/passes of the coating solution. Films in this example either included 20 or 30 passes as the first, outer polymer layer.

Then the second therapeutic solution was passed through a spray nozzle, to form droplets which fell from the tip of the nozzle onto the first polymer layer previously formed on the sheet of silicone. This formed a second layer which included both the polymer and therapeutic agent. The second layer included about 125 passes.

Then the first polymer solution was passed through the first spray nozzle again, creating a third, outer polymer layer. Droplets of the first polymer solution were formed and deposited onto the second layer previously formed on top of the first layer on the collagen sheet. The third outer layer also included 20 or 30 passes. A tri-layer structure was created having a first outer layer, a second layer, and a third outer layer, with a therapeutic drug loading of about 24 mg/mL.

The films were then soaked in Phosphate buffered saline (PBS) for up to 200 hours to determine drug release. Those results are shown in FIG. 11.

### EXAMPLE 6

A first polymer solution and a second polymer solution were provided. The first polymer solution contained 30 mg/mL poly(glycolide-co-caprolactone) dissolved in dichloromethane. The second polymer solution contained 50 mg/mL of bupivacaine hydrochloride and 30 mg/mL poly(glycolide-co-caprolactone) dissolved in dichloromethane and methanol (1:1 ratio).

The first and second solutions were fed into an ultrasonic sprayer and passed through an ultrasonic spray nozzle. The nozzle vibrated at a frequency of 48 kHz and was operated at 6 watts. The solutions were passed through the nozzles at a flow rate of about 2 mL/min while the nozzle was moving at a rate of 100 mm/sec at a height of 30 mm. A nitrogen curtain kept at a pressure of 1.0 kPa surrounded the falling droplets.

The first polymer solution was passed through a first spray nozzle to form droplets that were deposited onto a collagen sheet to form a first, outer polymer layer. The thickness of the polymer layer was controlled by the number of applications/passes of the coating solution. Films in this example included either 10, 20, 30, or 60 passes as the first, outer polymer layer.

A masking template was placed over the first, outer polymer layer. Then the second therapeutic and polymer solution were passed through a spray nozzle, to form droplets that fell from the tip of the nozzle onto the first polymer layer previously formed on the sheet of silicone. This formed a second layer which included both the polymer and therapeutic agent. The second layer included about 75 passes.

The masking template was removed. Then the first polymer solution was passed through the first spray nozzle again while the flow rate of the second therapeutic solution was stopped, creating a third, outer polymer layer. Droplets of the first polymer solution were formed and deposited onto the second layer previously formed on top of the first layer on the collagen sheet. The third outer layer also included 10, 20, 30, or 60 passes. A tri-layer structure was created having a first outer layer, a second layer, and a third outer layer.

The films were then soaked in PBS for up to 200 hours to determine drug release. Those results are shown in FIG. 12.

### EXAMPLE 7

A first polymer solution and a second polymer solution were provided. The first polymer solution contained 30 mg/mL poly(glycolide-co-caprolactone) dissolved in dichloromethane. The second polymer solution contained 30 mg/mL of bupivacaine and 50 mg/mL poly(glycolide-co-caprolactone) dissolved in dichloromethane and methanol (1:1 ratio).

The first and second solutions were fed into an ultrasonic sprayer and passed through an ultrasonic spray nozzle. The nozzle vibrated at a frequency of 48 kHz and was operated at 6 watts. The solutions were passed through the nozzles at a flow rate of about 2 mL/min while the nozzle was moving at a rate of 100 mm/sec at a height of 30 mm. A nitrogen curtain kept at a pressure of 1.0 kPa surrounded the falling droplets.

The first polymer solution was passed through a first spray nozzle to form droplets that were deposited onto a collagen sheet to form a first, outer polymer layer. The thickness of the polymer layer was controlled by the number of applications/passes of the coating solution. Films in this example included 30 passes as the first, outer polymer layers.

Then the second therapeutic solution was passed through a second spray nozzle, to form droplets which fell from the tip of the nozzle onto the first polymer layer previously formed on the sheet of silicone. This formed a second layer which included both the polymer and therapeutic agent. The second layer included about 75 passes.

Then the first polymer solution was passed through the first spray nozzle again, creating a third, outer polymer layer. Droplets of the first polymer solution were formed and deposited onto the second layer previously formed on top of the first layer on the collagen sheet. The third outer layer also included 30 passes. A tri-layer structure was created having a first outer layer, a second middle layer, and a third outer layer. The films illustrated in FIG. 10, PS33-4 and PS 36-5, were formulated to include 17.8 and 17.7 mg/cm² of bupivacaine hydrochloride, respectively.

The films were then soaked in PBS for up to 200 hours to determine drug release. Those results are shown in FIG. 10.

It will be understood that various modifications may be made to the embodiments disclosed herein. Therefore, the above description should not be construed as limiting, but merely as exemplifications within the scope and spirit of the claims appended hereto.

The present invention may also be described by reference to the following numbered paragraphs:-
1. A multilayer film comprising: a first layer comprising a first hydrophobic polymer; a third layer comprising a third hydrophobic polymer; and a second layer disposed between the first layer and the third layer, the second layer comprising a second hydrophobic polymer and at least one water-soluble therapeutic agent.
2. The multilayer film of paragraph 1, wherein at least one of the first, second, and third hydrophobic polymers is selected from the group consisting of aliphatic polyesters, polyhydroxyalkanoates, polyorthoesters, polyurethanes, polyanhydrides, polymer drugs, and combinations thereof.
3. The multilayer film of paragraph 2, wherein the aliphatic polyester is selected from the group consisting of lactide; glycolide; trimethylene carbonate; Ä-valerolactone; â-butyrolactone; ã-butyrolactone; å-decalactone; hydroxybutyrate; hydroxyvalerate; 1,4-dioxepan-2-one; 1,5-dioxepan-2-one; 6,6-dimethyl- 1,4-dioxan-2-one; 2,5-diketomorpholine; pivalolactone; á, á diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and homopolymers and copolymers and combinations thereof.
4. The multilayer film of paragraph 1, wherein the hydrophobic polymer of at least one of the first, second, and third layers comprises a copolymer of about 10 weight % glycolide and about 90 weight % ε -caprolactone.
5. The multilayer film of paragraph 1, wherein the multilayer film is formed by spray coating.
6. The multilayer film of paragraph 5, wherein the spray coating comprises ultrasonic spray coating.
7. The multilayer film of paragraph 1, wherein the at least one water-soluble therapeutic agent is selected from the group consisting of bupivacaine, 5-fluorouracil, cisplatin, methotrexate, and capsaicin.
8. The multilayer film of paragraph 7, wherein the at least one water-soluble therapeutic agent is bupivacaine hydrochloride.
9. The multilayer film of paragraph 7, wherein the at least one water-soluble therapeutic agent has a therapeutic payload of from about 1 mg/cm² to about 50 mg/cm².
10. The multilayer film of paragraph 1, further comprising a mesh having a first surface and a second surface.
11. The multilayer film of paragraph 10, wherein the mesh is positioned between the first and the third layer.
12. The multilayer film of paragraph 10, wherein the first surface of the mesh is positioned adjacent at least one of the first and the third layers.
13. The multilayer film of paragraph 1, further comprising a collagen film.
14. A method of treating cancer selected from the group consisting of breast cancer, lung cancer, esophageal cancer, gastric cancer, colon cancer, stomach cancer, and brain cancer, utilizing the multilayer film of paragraph 1.
15. The method of paragraph 14, wherein the at least one water-soluble therapeutic agent is selected from the group consisting of 5-fluorouracil, cisplatin, and methotrexate.
16. A method of treating post-operative pain, wherein the post-operative pain is associated with a surgery selected from the group consisting of hernia repair, hysterectomy, thoracotomy, coronary artery bypass, hemorrhoidectomy, adhesiolysis, breast reconstruction, spine surgery, and joint replacement/repair, utilizing the multilayer film of paragraph 1.
17. The method of paragraph 16, wherein the at least one water-soluble therapeutic agent is selected from the group consisting of bupivacaine hydrochloride and capsaicin.
18. The multilayer film of paragraph 1, including a therapeutic payload of at least 300 mg/cm² of bupivacaine HCI.
19. A multilayer film composite comprising: a mesh comprising a collagen film; and a multilayer film including a first layer comprising a first hydrophobic polymer, a third layer comprising a third hydrophobic polymer, and a second layer disposed between the first layer and the third layer, the second layer comprising a second hydrophobic polymer and at least one water-soluble therapeutic agent.
20. The multilayer film composite of paragraph 19, wherein the mesh includes a first surface and a second surface.
21. A method of making a multilayer film composite comprising: providing a collagen film; providing a first solution containing at least one hydrophobic polymer; providing a second solution containing at least one water-soluble therapeutic agent; passing the first solution through a first spray nozzle to generate first droplets of the first solution; depositing the first droplets on the collagen film to form a first layer; passing the second solution through a second spray nozzle to generate second droplets of the second solution; and depositing the second droplets on the first layer to form a second layer; and drying the first and second layers to form a multilayer film composite.
22. The method of paragraph 21, further comprising: combining the multilayer film composite with a mesh.
23. The method of paragraph 22, further comprising: depositing the first droplets on the second layer to form a third layer.
24. The method of paragraph 22, wherein the drying step occurs at ambient temperature.

## Claims

1. A multilayer film comprising:
a first layer comprising a first hydrophobic polymer;
a third layer comprising a third hydrophobic polymer; and
a second layer disposed between the first layer and the third layer, the second layer comprising a second hydrophobic polymer and at least one water-soluble therapeutic agent.

2. The multilayer film of claim 1, wherein at least one of the first, second, and third hydrophobic polymers is selected from the group consisting of aliphatic polyesters, polyhydroxyalkanoates, polyorthoesters, polyurethanes, polyanhydrides, polymer drugs, and combinations thereof, preferably wherein the aliphatic polyester is selected from the group consisting of lactide; glycolide; trimethylene carbonate; Ä-valerolactone; â-butyrolactone; ã-butyrolactone; å-decalactone; hydroxybutyrate; hydroxyvalerate; 1,4-dioxepan-2-one; 1,5-dioxepan-2-one; 6,6-dimethyl- 1,4-dioxan-2-one; 2,5-diketomorpholine; pivalolactone; á, á diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and homopolymers and copolymers and combinations thereof.

3. The multilayer film of claim 1 or claim 2, wherein the hydrophobic polymer of at least one of the first, second, and third layers comprises a copolymer of about 10 weight % glycolide and about 90 weight % ε -caprolactone.

4. The multilayer film of any preceding claim, wherein the multilayer film is formed by spray coating, preferably wherein the spray coating comprises ultrasonic spray coating.

5. The multilayer film of any preceding claim, wherein the at least one water-soluble therapeutic agent is selected from the group consisting of bupivacaine, 5-fluorouracil, cisplatin, methotrexate, and capsaicin, preferably wherein the at least one water-soluble therapeutic agent is bupivacaine hydrochloride, and/or wherein the at least one water-soluble therapeutic agent has a therapeutic payload of from about 1 mg/cm² to about 50 mg/cm².

6. The multilayer film of any preceding claim, further comprising a mesh having a first surface and a second surface.

7. The multilayer film of claim 6, wherein the mesh is positioned between the first and the third layer; and/or the multilayer film of claim 6, wherein the first surface of the mesh is positioned adjacent at least one of the first and the third layers.

8. The multilayer film of any preceding claim, further comprising a collagen film.

9. The multilayer film of any preceding claim for use in a method of treating cancer wherein the cancer is selected from the group consisting of breast cancer, lung cancer, esophageal cancer, gastric cancer, colon cancer, stomach cancer, and brain cancer

10. The multilayer film of claim 9, wherein the at least one water-soluble therapeutic agent is selected from the group consisting of 5-fluorouracil, cisplatin, and methotrexate.

11. The multilayer film of any preceding claim for use in a method of treating post-operative pain wherein the post-operative pain is associated with a surgery selected from the group consisting of hernia repair, hysterectomy, thoracotomy, coronary artery bypass, hemorrhoidectomy, adhesiolysis, breast reconstruction, spine surgery, and joint replacement/repair.

12. The multilayer film of claim 11, wherein the at least one water-soluble therapeutic agent is selected from the group consisting of bupivacaine hydrochloride and capsaicin.

13. The multilayer film of any preceding claim, including a therapeutic payload of at least 300 mg/cm² of bupivacaine HCI.

14. A multilayer film composite comprising:
a mesh comprising a collagen film; and
a multilayer film including a first layer comprising a first hydrophobic polymer, a third layer comprising a third hydrophobic polymer, and a second layer disposed between the first layer and the third layer, the second layer comprising a second hydrophobic polymer and at least one water-soluble therapeutic agent.

15. The multilayer film composite of claim 14, wherein the mesh includes a first surface and a second surface.

16. A method of making a multilayer film composite comprising:
providing a collagen film;
providing a first solution containing at least one hydrophobic polymer;
providing a second solution containing at least one water-soluble therapeutic agent;
passing the first solution through a first spray nozzle to generate first droplets of the first solution;
depositing the first droplets on the collagen film to form a first layer;
passing the second solution through a second spray nozzle to generate second droplets of the second solution; and
depositing the second droplets on the first layer to form a second layer; and
drying the first and second layers to form a multilayer film composite.

17. The method of claim 16, further comprising combining the multilayer film composite with a mesh; and/or the method of claim 16, further comprising depositing the first droplets on the second layer to form a third layer; and/or the method of claim 16, wherein the drying step occurs at ambient temperature.
